# FASCICULE DE BREVET EUROPEEN

(11) **EP 0 860 412 B1**
(45) Date de publication et mention de la délivrance du brevet: **15.11.2000**
(21) Numéro de dépôt: 98400357.4
(22) Date de dépôt: 16.02.1998
(51) Int. Cl.: C07C 11/02, C07C 1/20

(54) **Procédé de production d'oléfine tertiaire par décomposition d'éther alkylique tertiaire**
Verfahren zur Herstellung von tertiären Olefinen durch Spaltung von tertiären Alkylethern
Process for the preparation of tertiary olefins by decomposition of tertiary alkyl ethers

(30) Priorité: 21.02.1997 FR 9702194
(43) Date de publication de la demande: 26.08.1998
(73) Titulaire: INSTITUT FRANCAIS DU PETROLE, 92852 Rueil-Malmaison Cedex (FR)
(72) Inventeur: Marion, Marie-Claire, 69100 Villeurbanne (FR); Coupard, Vincent, 69002 Lyon (FR); Forestiere, Alain, 69390 Vernaison (FR); Travers, Philippe, 92500 Rueil Malmaison (FR); Viltard, Jean-Charles, 26000 Valence (FR)
(74) Mandataire: Andréeff, François

(56) Documents cités:
- FR-A- 2 669 021
- US-A- 4 447 668
- G. MARCEGLIA ET AL: 'Snamprogetti's Associated Technologies' CHEMICAL ECONOMY & ENGINEERING REVIEW vol. 14, no. 6, pages 35 - 40

## Description

L'invention concerne un procédé de décomposition d'éther(s) alkylique(s) tertiaire(s) pour la production d'oléfine(s) tertiaire(s) de haute pureté. Elle concerne en particulier un procédé de production d'isobutène de très haute pureté, et de méthanol, à partir de l'éther méthylique de l'alcool tertiobutylique (MTBE initiales anglaises de MethylTertio-Butyl-Ether). Le procédé selon la présente invention s'applique à la synthèse de toute oléfine tertiaire à partir d'éther alkylique tertiaire [par exemple ETBE (éther éthylique de l'alcool tertiobutylique des initiales anglaises Ethyl-Tertio-Butyl-Ether), ETAE (éther méthylique de l'alcool tertioamylique des initiales anglaises Tertio-Amyl-Methyl-Ether), TAME, isopropyl-tertio-butyl-éther]. La suite de la description, et tout particulièrement les conditions opératoires, sont données à titre indicatif pour la synthèse d'isobutène à partir de MTBE.

Il existe diverses voies de production d'isobutène de haute pureté exploitées industriellement. La plus ancienne est le procédé par extraction à l'acide sulfurique, mais il est cher et obsolète ; il est réputé polluant car il produit des rejets d'acide usé. De plus, le rendement en isobutène ne dépasse pas 90 %. La firme ARCO utilise la voie par déshydratation de l'alcool tertio-butylique (ABT), ce dernier étant un sous-produit obtenu avec leur procédé de production d'oxyde de propylène. Le procédé par déshydrogénation de l'isobutane s'est développé au cours de ces dernières années par suite de la demande importante et croissante en MTBE. Toutefois, ce procédé n'est mis en oeuvre de façon rentable que pour de très grosses capacités de production.

La production d'isobutène de haute pureté par craquage du MTBE est aussi bien adaptée pour des petites capacités que pour des grosses capacités. De plus, cette voie bénéficie de toute l'infrastructure relative à l'importance croissante des éthers dans les essences reformulées. De nombreuses raffineries, partout dans le monde, possèdent des installations de productions de MTBE par exemple. D'autre part il y a également un marché d'échange du MTBE au niveau mondial. Cela signifie que la production d'isobutène de haute pureté, à partir de MTBE, peut être mise en oeuvre aisément partout dans le monde, y compris en dehors des raffineries.

L'idée de produire de l'isobutène par décomposition d'éther, et plus particulièrement du MTBE, est connue depuis longtemps, mais les procédés de mise en oeuvre proposés par l'art antérieur présentent certains inconvénients.

Ainsi dans le procédé développé par SUMITOMO, décrit par exemple dans la demande de brevet EP-A-68 785, la réaction de décomposition du MTBE est réalisée en phase liquide, en présence d'un catalyseur solide acide de type résine échangeuse d'ions. Deux flux de produit sont obtenus : l'isobutène et le méthanol. Tel que le schéma est décrit, l'isobutène est directement obtenu en tête d'une colonne à distiller sans autre étape de purification. L'isobutène ainsi obtenu contient un certain nombre d'impuretés à commencer par une petite fraction de méthanol qui est distillée par azéotropie, du diméthyléther (DME) composé volatil formé par condensation du méthanol en présence d'un catalyseur acide. Il est probable que la pureté de l'isobutène soit alors insuffisante pour une utilisation telle que la fabrication de polyisobutène ou d'autres copolymères. En outre, aucun moyen ne permet apparemment d'éviter l'accumulation d'impuretés lourdes telles que les dimères de l'isobutène ou l'éther méthylique de l'alcool butylique secondaire (MSBE), qui à terme se traduit fatalement par une baisse de pureté des produits.

Dans le procédé développé par ERDOLCHEMIE, décrit par exemple dans le brevet US-A-4 409 421 la purification de l'isobutène, qui consiste à éliminer l'alcool résiduel entraîné avec l'oléfine tertiaire, est réalisée par adsorption. Cette méthode, présente l'inconvénient d'avoir à régénérer régulièrement l'adsorbant. En outre la récupération de la majeure partie de l'alcool issu de la décomposition n'est pas solutionnée.

Plus récemment, la même société décrit, dans le brevet US-A-5 095 164 la mise en oeuvre de la réaction de décomposition dans un appareillage de distillation. Le catalyseur est alors placé dans le fond de la colonne au niveau du rebouilleur. Cette mise en oeuvre particulière est limitative sur le plan de la température réactionnelle, directement imposée par la nature de l'éther et la pression opératoire. En outre, elle favorise vraisemblablement la formation de sous-produits de réaction tels que la formation de dimères de l'isobutène et/ou la formation de diméthyléther. A ce propos, la qualité et/ou le devenir des produits n'est pas clairement explicité.

Dans le brevet US-A-4 447 668 on décrit un procédé de préparation d'oléfines par décomposition d'éthers; une étape de séparation par lavage et distillation est mentionnée.

Par ailleurs, la société BASF décrit, dans le brevet US-A-4 287 379, un schéma intégrant à la fois l'étape de synthèse de l'éther sa séparation puis l'étape de décomposition de l'éther pour produire l'isobutène. Toutefois, pour éviter certaines étapes de purification, l'éthérification est faite avec un alcool en C3 ou C4, ce qui est un inconvénient majeur face au marché international du MTBE.

Enfin, nous pouvons encore citer les deux schémas du procédé SNAMPROGETTI présentés dans Chemical Economy & Engineering Review, vol. 14 n° 6 Juin 1982, incluant à la fois l'étape de synthèse du MTBE et l'étape de décomposition du MTBE pour la production d'isobutène. Il apparaît dans ces schémas qu'une certaine perte d'eau par entraînement et/ou saturation du flux isobutène, au niveau du lavage de l'isobutène pour éliminer l'alcool, n'est pas prise en compte. Cela peut se traduire à terme soit par une baisse du débit d'eau de lavage, soit par une perte d'efficacité de cette section de lavage. Cela peut nuire alors à la qualité de l'isobutène produit. Par ailleurs selon ces schémas la fraction hydrocarbonée issue de la colonne d'extraction à l'eau qui contient une quantité relativement importante d'eau libre est envoyée en totalité dans la colonne de fractionnement permettant de récupérer l'isobutène purifié ce qui implique que cette colonne doit traiter une quantité de produit importante et donc avoir des caractéristiques de dimensionnement importante ce qui rend le procédé particulièrement onéreux et délicat à mettre en oeuvre.

Le procédé selon l'invention permet de remédier aux inconvénients précités. Il concerne un procédé de production d'oléfine(s) tertiaire(s) caractérisée(s) par une très haute pureté, à partir d'un éther alkylique tertiaire.

L'invention concerne un procédé de décomposition d'éther(s) alkylique tertiaire(s), en particulier tel(s) que défini(s) précédemment, de préférence de MTBE ou ETBE, pour la production d'oléfine(s) tertiaire(s), d'isobutène en particulier, de haute pureté. Dans le cas de la décomposition d'autres éthers, on peut obtenir un mélange contenant une pluralité d'oléfines tertiaires. Ainsi dans le cas de la décomposition du TAME on obtient un mélange contenant du méthyl-2 butène-1 et du méthyl-2 butène-2.

Outre la zone de réaction proprement dite, le procédé selon l'invention comprend des zones de purification ou récupération ou recyclage des divers produits de façon à optimiser la valorisation des produits mis en oeuvre et minimiser les pertes.

La présente invention concerne un procédé de production d'oléfine tertiaire par décomposition d'éther alkylique tertiaire comprenant :
a) une étape de décomposition d'au moins un éther alkylique tertiaire dans une zone réactionnelle comprenant au moins un réacteur (R1) contenant un catalyseur de décomposition dudit éther, ladite étape étant effectuée dans des conditions permettant la décomposition au moins partielle dudit éther alkylique tertiaire en un produit contenant au moins un alcool et au moins une oléfine tertiaire,
b) une étape de fractionnement d'au moins une partie du produit issu de l'étape a), et de préférence de la totalité de ce produit, dans une zone de fractionnement (C1) permettant d'obtenir d'une part une fraction (A) contenant la majeure partie de l'oléfine tertiaire et éventuellement une fraction mineure d'alcool et des composés légers éventuels généralement contenus initialement dans le produit issu de l'étape a), et d'autre part une fraction (B) contenant la majeure partie de l'alcool formé dans l'étape a) et éventuellement de l'éther non décomposé dans l'étape a),
c) une étape de purification d'au moins une partie de la fraction (A) dans laquelle ladite partie est envoyée dans une zone d'extraction (L1) par lavage à l'eau à partir de laquelle on obtient une fraction aqueuse (C) contenant la majeure partie de l'alcool initialement présent dans ladite partie et une fraction (D) contenant la majeure partie de l'oléfine tertiaire initialement présente dans ladite partie, ladite fraction (D) contenant ladite oléfine tertiaire, de l'eau, éventuellement des composés légers et étant sensiblement exempte d'alcool,
ledit procédé étant caractérisé en ce qu'il comporte une étape d) dans laquelle au moins une partie de la fraction (D) issue de l'étape c) est envoyée dans une zone de séparation (Co) à partir de laquelle on récupère une fraction liquide aqueuse (Le) et une fraction liquide (Lc) contenant la majeure partie de l'oléfine tertiaire initialement présente dans la fraction (D), ladite fraction (Lc) contenant ladite oléfine tertiaire, une faible quantité d'eau et éventuellement des composés légers.

Dans une forme particulière de réalisation du procédé selon l'invention, permettant généralement d'obtenir une oléfine tertiaire de pureté élevée, au moins une partie de la fraction liquide (Lc) récupérée à l'étape d) est envoyée dans une étape e) dans une zone de fractionnement (C2) dans laquelle ladite partie de la fraction (Lc) est fractionnée d'une part en une fraction (E) contenant l'oléfine tertiaire et d'autre part en une fraction (F) contenant la majeure partie des composés légers éventuels et éventuellement une faible quantité d'eau résiduelle. La fraction (F) peut être scindée en une fraction gazeuse que l'on évacue par exemple à la torche et en une fraction liquide qui est au moins en partie renvoyée dans la zone de fractionnement (C2) de l'étape e) [ligne (20b) issue de la ligne (20) et/ou de la ligne (22)].

Selon cette forme particulière il est habituellement préférable que la zone de fractionnement de l'étape e) comporte au moins un moyen permettant de récupérer à partir de la fraction (F) une fraction légère sensiblement anhydre. Le plus souvent ce moyen permettra de scinder au moins une partie de la fraction (F) en une fraction légère sensiblement anhydre et en une fraction aqueuse. Ce moyen sera par exemple un ballon séparateur muni au moins d'au moins un moyen, par exemple une botte, permettant la décantation et le soutirage d'une fraction aqueuse. Dans ce cas au moins une partie de la fraction aqueuse obtenue à l'étape e) est de préférence recyclée à l'étape c) dans la zone (L1) d'extraction par lavage à l'eau. Alors, la fraction légère sensiblement anhydre est habituellement scindée en une fraction gazeuse que l'on évacue par exemple à la torche et en une fraction liquide généralement sensiblement anhydre qui est au moins en partie renvoyée dans la zone de fractionnement (C2) de l'étape e). Selon un autre mode de mise en oeuvre la fraction (F) [ou la fraction légère obtenue à partir de ladite fraction (F)] qui est issue de l'étape e) est au moins en partie envoyée dans une zone de craquage catalytique. Selon une autre variante, la fraction (F) [ou la fraction légère obtenue à partir de ladite fraction (F)], qui est issue de l'étape e), est au moins en partie envoyée dans une zone de synthèse d'éther par réaction entre au moins une oléfine tertiaire et au moins un alcool. Selon encore une autre variante, la fraction (F) [ou fraction légère obtenue à partir de ladite fraction (F)], qui est issue de l'étape e), est au moins en partie envoyée à la torche.

Le plus souvent le procédé de la présente invention comprend une étape f) dans laquelle au moins une partie de la fraction aqueuse (C) issue de l'étape c) est envoyée dans une zone de fractionnement (C3) à partir de laquelle on récupère une fraction (G) contenant la majeure partie de l'alcool initialement présent dans ladite partie et une fraction aqueuse (H) débarrassée de la majeure partie de l'alcool initialement présent dans ladite partie. Selon ce mode de fonctionnement, au moins une partie de la fraction (G) obtenue à l'étape f) contenant de l'alcool peut être envoyée dans une zone de synthèse d'éther par réaction entre au moins une oléfine tertiaire et au moins un alcool. Il est également possible d'envoyer la totalité de cet alcool dans ladite zone de synthèse d'éther. On peut également récupérer en partie ou en totalité cet alcool pour d'autres usages. Selon ce mode de fonctionnement, au moins une partie de la fraction aqueuse (H) obtenue à l'étape f) peut aussi être au moins en partie recyclée à l'étape c) dans la zone (L1) d'extraction par lavage à l'eau. Selon ce mode de fonctionnement, au moins une partie de la fraction aqueuse (H) obtenue à l'étape f) peut aussi être au moins en partie envoyée dans une section de traitement des eaux.

Dans une forme préférée de mise en oeuvre du procédé de la présente invention au moins une partie de la fraction (B) obtenue à l'étape b), contenant la majeure partie de l'alcool formé dans l'étape a) et éventuellement de l'éther non décomposé dans l'étape a), peut être envoyée dans une zone de synthèse d'éther par réaction entre au moins une oléfine tertiaire et au moins un alcool. Il est également possible d'envoyer la totalité de cette fraction dans ladite zone de synthèse d'éther. On peut également récupérer en partie ou en totalité cette fraction pour d'autres usages.

Dans une forme préférée de mise en oeuvre du procédé de la présente invention, au moins une partie de la fraction aqueuse (Le) obtenue à l'étape d) est recyclée à l'étape c) dans la zone d'extraction (L1) par lavage à l'eau.

Ces divers recyclages d'eau sont indépendants les uns des autres et peuvent être réalisés ensembles ou séparément. L'eau qui n'est pas recyclée est généralement purgée puis habituellement envoyée vers une zone de traitement des eaux usées. Cette purge est le plus souvent présente au moins sur la fraction aqueuse (H) obtenue à l'étape f). Cette purge permet en particulier d'éviter une éventuelle accumulation de composés dits lourds, par exemple d'alcools lourds.

Les conditions de mise en oeuvre de l'étape a) de la présente invention sont des conditions classiques de décomposition d'éther alkylique tertiaire bien connues de l'homme du métier. Dans une forme préférée de réalisation cette étape a) sera mise en oeuvre sans addition d'eau supplémentaire au produit introduit dans la zone de décomposition. Il serait cependant possible d'ajouter une certaine quantité d'eau par exemple jusqu'à la limite de solubilité de l'eau dans l'éther que l'on souhaite décomposer. Habituellement les conditions de mise en oeuvre de cette étape a) sont choisies de manière à ce que la majeure partie de l'éther alkylique tertiaire se décompose pour donner un alcool et une oléfine tertiaire. Dans cette zone de décomposition la pression absolue est habituellement d'environ 1 à environ 12 bar ( 1 bar est égal à 0,1 MPa), la température est habituellement comprise entre 50 °C et 300 °C et de préférence entre 100 °C et 250 °C, la VVH (vitesse spatiale horaire) est habituellement comprise entre 0,1 et 200 h⁻¹ et le plus souvent entre 0,5 et 100 h⁻¹. Dans cette zone on peut utiliser tous les catalyseurs acides bien connus de l'homme du métier. On préfère habituellement employer des catalyseurs solides acides. Ainsi le catalyseur peut être choisi dans le groupe formé par les résines acides organiques et les résines acides minérales, généralement solides dans les conditions de la réaction de décomposition dudit éther. Parmi ces composés, on emploie le plus souvent ceux choisis dans le groupe formé par les solides minéraux greffés comportant au moins un groupe organique de type alkyl-sulfonique, aryl-sulfonique ou alkylaryl-sulfonique. L'une des formes préférée de mise en oeuvre de cette étape a) utilise un catalyseur choisi dans le groupe formé par les polysiloxanes greffés par au moins un groupe alkyl-sulfonique.

Les conditions générales de mise en oeuvre de l'étape b) de fractionnement du produit issu de l'étape a) de décomposition de l'éther est une étape dont les conditions sont choisies en particulier en fonction des caractéristiques de l'alcool et de l'oléfine tertiaire formés. L'homme du métier est à même de choisir ces conditions pour obtenir la séparation souhaitée entre une fraction contenant la majeure partie de l'alcool et une fraction contenant la majeure partie de l'oléfine. Ainsi par exemple dans le cas de la décomposition du MTBE et de la formation de méthanol et d'isobutène la pression absolue dans la colonne de distillation est d'environ 1 à environ 15 bar, de préférence d'environ 1 à environ 10 bar, identique ou différente de celle régnant dans la zone de décomposition. La température de fond de la colonne dépend à la fois de la pression régnant dans ladite colonne et de la composition du produit de fond, en particulier du rapport molaire entre le méthanol et le MTBE éventuellement présent par suite d'une décomposition partielle de cet éther dans l'étape a). Dans le cas d'une unité traitant 1 kg/h de MTBE la colonne de distillation comporte habituellement entre 3 et 80 plateaux théoriques et le plus souvent entre 10 et 50 plateaux théoriques.

Dans l'étape c) de purification d'au moins une partie de la fraction (A), ladite partie de la fraction est envoyée dans une zone d'extraction (L1) par lavage à l'eau ; la quantité d'eau utilisée pour ce lavage est habituellement telle que le rapport volumique entre le volume de ladite quantité d'eau introduite dans ladite zone d'extraction et celui de ladite partie de fraction (A) introduite dans ladite zone d'extraction (Vₑₐᵤ/V_{A}) est d'environ 0,005 à environ 20. Le plus souvent cette quantité d'eau est telle que le rapport Vₑₐᵤ/V_{A} est d'environ 0,005 à environ 10, de préférence d'environ 0,01 à environ 5 et de manière encore plus préférée d'environ 0,02 à environ 1. Le débit d'eau dans cette zone de lavage (L1) est très souvent régulé en fonction du maintien d'un niveau de fond dans la zone de fractionnement (C3) de l'eau et de l'alcool dans le cas de la présence d'une telle zone (C3). Ce niveau de fond peut être défini comme le niveau minimal nécessaire au bon fonctionnement de ladite zone (C3). Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglo-saxonnes Level Control Regulation. Quel que soit le mode de régulation choisi, la quantité d'eau peut être généralement ajustée à l'aide d'un moyen d'introduction d'eau d'appoint dans la zone (L1). Cet appoint d'eau permet en particulier de compenser les pertes d'eau dues à l'entraînement d'eau et/ou à la saturation du flux hydrocarboné traité ainsi que le remplacement de l'eau éventuellement purgée. Cette zone d'extraction (L1) est habituellement une colonne à plateaux qui opère à une température d'environ 1 à environ 100 °C de préférence d'environ 10 à environ 60 °C. La pression absolue dans cette zone sera d'environ 1 à environ 20 bar, le plus souvent d'environ 1 à environ 15 bar, identique ou différente de celle régnant dans la zone de fractionnement de l'étape b).

L'étape d) qui comprend une zone de séparation d'au moins une partie de la fraction (D) en une fraction liquide aqueuse (Le) et en une fraction liquide hydrocarbonée (Lc) dans une zone (Co), est une étape classique bien connue de l'homme du métier. Cette étape est habituellement mise en oeuvre dans un appareil dénommé coalesceur, dans lequel l'eau se rassemble à la partie inférieure de l'appareil par coalescence. Les conditions de température et de pression régnant dans cette zone sont dans les mêmes gammes que celles régnant dans l'étape c) d'extraction à l'eau. La pression (respectivement la température) peut être identique ou différente de celle régnant dans la zone (L1) l'étape c). Dans cette zone on sépare ainsi l'eau libre contenue dans le produit issu de l'étape c). De plus cette zone a également le plus souvent une fonction de zone ou ballon de charge pour la zone de purification (C2) de l'isobutène, dans le cas de la présence d'une telle zone (C2). Tout autre moyen connu de l'homme du métier peut être employé dans le cadre de la présente invention. A titre d'exemple, on peut citer l'utilisation d'un absorbant ayant une sélectivité préférentielle pour l'une des fractions aqueuse ou organique.

L'étape e) éventuelle de fractionnement de la fraction (Lc) dans une zone (C2) en une fraction (E) contenant l'oléfine tertiaire et en une fraction (F) contenant la majeure partie des composés légers éventuellement présents dans ladite fraction (Lc) et l'eau résiduelle contenue dans la fraction liquide (Lc), est habituellement effectuée dans une colonne à distiller fonctionnant sous une pression absolue d'environ 1 à environ 15 bar, le plus souvent d'environ 3 à environ 10 bar, identique ou différente de celle régnant dans la zone de séparation de l'étape d). Pour une unité produisant 0,6 kg/h d'isobutène cette colonne a habituellement environ 3 à environ 80 plateaux théoriques et le plus souvent environ 5 à environ 50 plateaux théoriques. La température de fond de la colonne dépend en particulier de la pression régnant dans ladite colonne.

L'étape f) éventuelle de fractionnement, dans une zone (C3), de la fraction aqueuse (C), contenant la majeure partie de l'alcool initialement présent dans la fraction (A), en une fraction (G) contenant la majeure partie de l'alcool initialement présent dans la fraction (C) et en une fraction aqueuse (H) débarrassée de la majeure partie de l'alcool initialement présent dans la fraction (C), est habituellement réalisée dans une colonne à distiller (C3) sous une pression absolue d'environ 1 à environ 12 bar, de préférence d'environ 1 à environ 8 bar, identique ou différente de celle régnant dans la zone d'extraction à l'eau de l'étape c). La température de fond de la colonne dépend en particulier de la pression régnant dans ladite colonne; elle est habituellement d'environ 50 à environ 300 °C et le plus souvent d'environ 65 à environ 200 °C. La colonne comporte habituellement environ 2 à environ 80 plateaux et le plus souvent environ 3 à environ 60 plateaux théoriques.

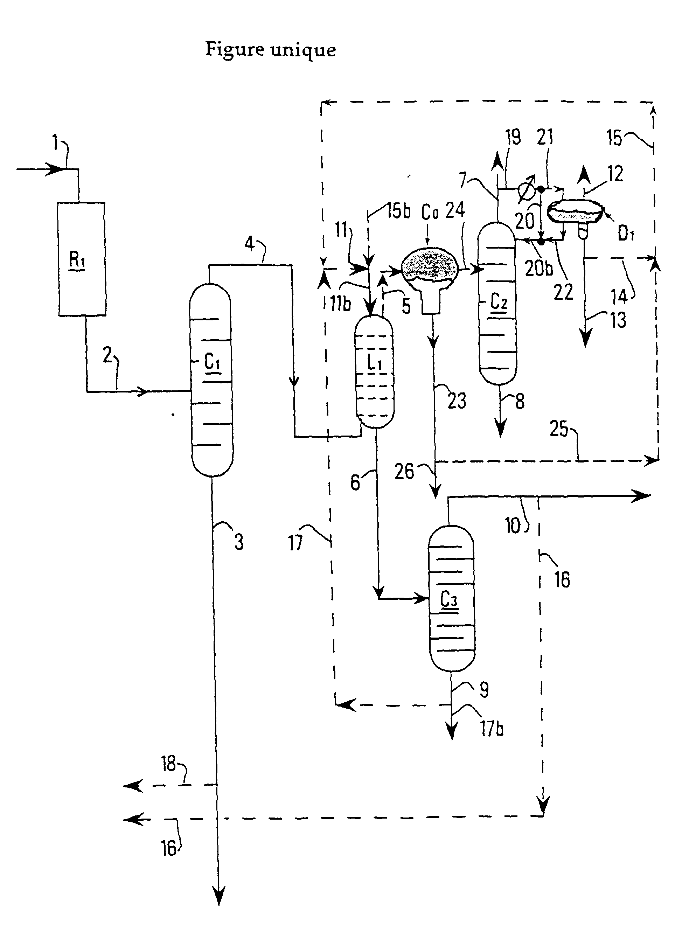

La figure 1 est un schéma de principe illustrant l'une des variantes préférées de mise en oeuvre de la présente invention. Les traits en pointillés montrent les diverses options possibles.

La charge contenant l'éther alkylique tertiaire est introduite par la ligne 1 dans le réacteur (R1) de décomposition dudit éther. Ce réacteur contient un catalyseur acide. Le produit de décomposition sort du réacteur (R1) et est envoyé dans une colonne de fractionnement (C1) par la ligne 2 à partir de laquelle on récupère par la ligne 4 un produit contenant une oléfine tertiaire et par la ligne 3 un produit contenant un alcool et le cas échéant de l'éther non décomposé. Le produit sortant de la colonne (C1) par la ligne 3 peut par exemple être en partie envoyé par la ligne 18 vers une zone de synthèse d'éther alkylique tertiaire. Le produit contenant l'oléfine tertiaire est introduit par la ligne 4 dans une zone d'extraction à l'eau (L1) dans laquelle de l'eau est introduite par la ligne 11b et à partir de laquelle on récupère une fraction (D) appauvrie en alcool par la ligne 5 que l'on envoie dans la zone de séparation Co à partir de laquelle on récupère par la ligne 23 une fraction liquide aqueuse (Le) et par la ligne 24 une fraction liquide hydrocarbonée (Lc) contenant la majeure partie de l'oléfine tertiaire initialement présente dans la fraction (D), ladite fraction (Lc) contenant ladite oléfine tertiaire, une faible quantité d'eau et éventuellement des composés légers étant envoyée par la ligne 24 dans une zone de fractionnement (C2). Par la ligne 6 on récupère un produit aqueux contenant de l'alcool que l'on introduit dans la zone de fractionnement (C3). A partir de la zone de fractionnement (C2) on récupère par la ligne 8 de l'oléfine tertiaire le plus souvent ultra pure et par la ligne 7 des produits légers qui sont par exemple en partie envoyés à la torche, mais qui peuvent aussi être envoyés dans une zone de craquage catalytique ou bien dans une zone de synthèse d'éther, et en partie recyclés à titre de reflux par les lignes 19, 20 et 20b dans la zone de fractionnement (C2). Il est aussi possible, dans une forme préférée de réalisation, d'envoyer au moins une partie de ces produits légers par les lignes 19 et 21 dans une zone de séparation (D1) à partir de laquelle on récupère par la ligne 13 une fraction constituée en majeure partie d'eau, par la ligne 22 une fraction liquide de produits légers que l'on renvoie à titre de reflux dans la colonne (C2) par la ligne 20b ; on récupère par la ligne 12 au moins une partie des produits légers gazeux qui sont par exemple en partie envoyés à la torche, mais qui peuvent aussi être envoyés dans une zone de craquage catalytique ou bien dans une zone de synthèse d'éther. Il est encore possible de combiner les deux formes de réalisation décrites précédemment. La fraction aqueuse peut être récupérée par la ligne 13 ou peut être par exemple renvoyée en partie par les lignes 14,15, et 11 11b dans la zone d'extraction à l'eau L1, en plus d'un apport éventuel extérieur en eau par la ligne 15b. A partir de la zone de fractionnement (C3) on récupère par la ligne 10 de l'alcool qui peut par exemple en partie être envoyé par la ligne 16 vers une zone de synthèse d'éther alkylique tertiaire. A partir de cette zone (C3), on récupère également une fraction aqueuse par la ligne 9 qui peut être envoyée au moins en partie vers une zone de traitement des eaux par la ligne 17b ou recyclée par les lignes 17, 11 et 11b au moins en partie dans la zone d'extraction à l'eau (L1). La fraction liquide aqueuse (Le) récupérée par la ligne 23 à partir de la zone de séparation (Co) peut être envoyée au moins en partie vers une zone de traitement des eaux par la ligne 26 ou recyclée par les lignes 25, 15, 11 et 11b au moins en partie dans la zone d'extraction à l'eau (L1).

L'exemple suivant illustre l'invention sans en limiter la portée.

### Exemple 1

On utilise un équipement de type pilote comprenant un réacteur tubulaire (R1), de 10 millilitres de volume, fonctionnant sous une pression relative de 7 bar, à une température moyenne de 160 °C contient 3 grammes de catalyseur de type polysiloxane greffé par des groupements alkyl sulfonique. On utilise un catalyseur commercial à base de polysiloxanes greffés par au moins un groupe alkyl-sulfonique. On alimente le réacteur (R1) par une charge contenant 100 % poids de MTBE, sous une VVH de 15 h⁻¹. Le tableau 1 présente la composition de la charge introduite dans le réacteur R1 de décomposition du MTBE et la composition du produit recueilli à la sortie du réacteur (R1).

**Tableau 1**

| | Charge(% poids) | Effluent R1 (% poids) |
|---|---|---|
| MTBE | 100 | 10 |
| Isobutène | | 56,1 |
| Méthanol | | 32,1 |
| DME | | 0,5 |
| Dimères | | 1,1 |
| H₂O | | 0,2 |

A l'aide du logiciel commercialisé par la société américaine SIMSCI (SIMulation SClence INC.) sous la dénomination commerciale Pro II, on calcule les diverses sections de purification.

Une colonne de distillation (C1), fonctionnant sous une pression relative de 7 bar et comportant 10 plateaux théoriques, est utilisée à l'étape b) du procédé de l'invention pour obtenir un produit de fond (B) et un produit de tête (A).

Une colonne d'extraction par lavage à l'eau L1, qui est une colonne à plateaux et qui fonctionne à température 30 °C sous une pression relative de 12 bar, est utilisée dans l'étape c) du procédé de l'invention pour obtenir une fraction aqueuse (C) et une fraction organique (D).

Un système d'extraction de l'eau libre entraînée à l'étape c) dans la fraction (D), du type coalesceur (Co), permet d'obtenir une fraction aqueuse (Le) et une fraction organique (Lc). Il fonctionne sous une pression relative de 12 bar à une température de 30 °C.

Une colonne de distillation (C2), dernière étape de purification de l'isobutène, fonctionnant sous une pression relative de 7 bar, et comportant 10 plateaux théoriques, est utilisée à l'étape e) pour obtenir un produit de fond (E) qui est de l'isobutène purifié et un produit de tête (F) contenant des composés légers.

La colonne (C1) est alimentée par l'effluent de (R1). Le produit (A) recueilli en tête de (C1) est envoyé dans la colonne d'extraction (L1) où il est lavé par une quantité d'eau dont le débit volumique est égal au dixième du débit de (A). On recueille une fraction aqueuse (C) contenant la majeure partie du méthanol contenu dans (A), et une fraction hydrocarbonée (D) contenant une petite quantité d'eau libre entraînée. Cette fraction d'eau libre est enfin éliminée après décantation dans le système de décantation (Co) sous forme d'une fraction (Le) et on récupère une fraction organique hydrocarbonée (Lc) . Enfin, la fraction hydrocarbonée (Lc) est traitée dans la colonne (C2) afin de produire en fond de colonne une fraction (E) qui est de l'isobutène de haute pureté et en tête une fraction légère (F) contenant en particulier le diméthyl éther (DME).

Les bilans matières obtenus sont donnés dans le tableau 2 et le tableau 3 ci-après.

**Tableau 2**

| | Effluent du réacteur (R1) (% poids) | Produit (B) colonne (C1) (g/h) | Produit (A) colonne (C1) (g/h) | Eau de lavage colonne (L1) | fraction (C) colonne (L1) | fraction (D) colonne (L1) |
|---|---|---|---|---|---|---|
| MTBE | 10 | 10 | | | | |
| Isobutène | 56,1 | | 56,1 | | | 56,1 |
| Méthanol | 32,1 | 30,4 | 1,7 | | 1,7 | - |
| DME | 0,5 | | 0,5 | | | 0,5 |
| Dimères | 1,1 | 1,1 | | | | - |
| H2O | 0,2 | | 0,2 | 10 | 9,2 | 1 |
| Débit (g/h) | 100 | 41,5 | 58,5 | 10 | 10,9 | 57,6 |

**Tableau 3**

| | fraction aqueuse (Le) extraite par (Co) | fraction organique (Lc) sortie de (Co) | fraction (F) colonne (C2) | fraction (E) colonne (C2) |
|---|---|---|---|---|
| Isobutène | | 56,1 | 1,72 | 54,38 |
| DME | | 0,5 | 0,49 | 0,01 |
| H2O | 0,99 | 0,01 | 0,01 | |
| Débit (g/h) | 0,99 | 56,61 | 2,22 | 54,39 |
| Pureté de l'isobutène (%) | | | | 99,9 % |
| Rendement en isobutène (%) | | | | 96 % |

Ainsi le procédé selon l'invention permet d'obtenir une pureté d'isobutène très élevée.

### Exemple 2

Soit un équipement de type pilote comprenant un réacteur tubulaire R1, fonctionnant sous une pression relative de 7 bar, à une température moyenne de 140 °C. Le réacteur R1 contient du Deloxan ASP (catalyseur de type polysiloxane greffé par des groupements alkyl sulfonique). On alimente R1 par une charge contenant 100 % poids de TAME, sous une VVH de 6 h-1. Le produit recueilli à la sortie de R1 possède la composition donnée dans le tableau 4 :

**Tableau 4 :**

| Section réactionnelle de décomposition du MTBE | | |
|---|---|---|
| | Charge (% poids) | Effluent R1 (% poids) |
| TAME | 100 | 15 |
| Isoamylènes | | 58,2 |
| Méthanol | | 26,45 |
| DME | | 0,25 |
| Dimères | | 0,1 |

A l'aide du logiciel Pro II, les divers sections de purification sont calculés comme dans l'exemple précédent.

Un bilan matière sommaire est donné dans le tableau 5 à titre d'illustration.

**Tableau 5**

| | Effluent R1 (% poids) | Tête de C1 (g/h) | Fond de C1 (g/h) | Fraction organique D après lavage (g/h) | Fraction Lc sortie de Co (g/h) |
|---|---|---|---|---|---|
| TAME | 15 | | 15 | | |
| Isoamylènes | 58,2 | 58,2 | | 58,2 | 58,2 |
| Méthanol | 26,45 | 2 | 24,45 | | |
| DME | 0,25 | 0,25 | | 0,25 | 0,25 |
| Dimères | 0,1 | | 0,1 | | |
| H2O | | | | 1,55 | 0,01 |
| Débit (g/h) | 100 | 60,45 | 39,55 | 60 | 58,46 |

On produit ainsi les isoamylènes avec un rendement minimum de 84 % (rendement pouvant être amélioré par le biais de recyclage de l'éther non converti) et avec une pureté supérieur à 99 %.

## Revendications

1. Procédé de production d'oléfine tertiaire par décomposition d'éther alkylique tertiaire comprenant :
a) une étape de décomposition d'au moins un éther alkylique tertiaire dans une zone réactionnelle comprenant au moins un réacteur (R1) contenant un catalyseur de décomposition dudit éther, ladite étape étant effectuée dans des conditions permettant la décomposition au moins partielle dudit éther alkylique tertiaire en un produit contenant au moins un alcool et au moins une oléfine tertiaire,
b) une étape de fractionnement d'au moins une partie du produit issu de l'étape a) dans une zone de fractionnement (C1) permettant d'obtenir d'une part une fraction (A) contenant la majeure partie de l'oléfine tertiaire et éventuellement une fraction mineure d'alcool et des composés légers éventuels, et d'autre part une fraction (B) contenant la majeure partie de l'alcool formé dans l'étape a) et éventuellement de l'éther non décomposé dans l'étape a),
c) une étape de purification d'au moins une partie de la fraction (A) dans laquelle ladite partie est envoyée dans une zone d'extraction (L1) par lavage à l'eau à partir de laquelle on obtient une fraction aqueuse (C) contenant la majeure partie de l'alcool initialement présent dans ladite partie et une fraction (D) contenant la majeure partie de l'oléfine tertiaire initialement présente dans ladite partie, ladite fraction (D) contenant ladite oléfine tertiaire, de l'eau, éventuellement des composés légers et étant sensiblement exempte d'alcool,
ledit procédé étant caractérisé en ce qu'il comporte une étape d) dans laquelle au moins une partie de la fraction (D) issue de l'étape c) est envoyée dans une zone de séparation (Co) à partir de laquelle on récupère une fraction liquide aqueuse (Le) et une fraction liquide (Lc) contenant la majeure partie de l'oléfine tertiaire initialement présente dans la fraction (D), ladite fraction (Lc) contenant ladite oléfine tertiaire, une faible quantité d'eau et éventuellement des composés légers.

2. Procédé selon la revendication 1 dans lequel au moins une partie de la fraction liquide (Lc) récupérée à l'étape d) est envoyée dans une étape e) dans une zone de fractionnement (C2) dans laquelle ladite partie de la fraction (Lc) est fractionnée d'une part en une fraction (E) contenant l'oléfine tertiaire et d'autre part en une fraction (F) contenant la majeure partie des composés légers éventuels et éventuellement une faible quantité d'eau résiduelle.

3. Procédé selon la revendication 2 dans lequel la zone de fractionnement de l'étape e) comporte au moins un moyen permettant de récupérer à partir de la fraction (F) une fraction légère sensiblement anhydre.

4. Procédé selon la revendication 3 dans lequel ledit moyen comprend un ballon séparateur muni d'au moins un moyen permettant la décantation et le soutirage d'une fraction aqueuse.

5. Procédé selon la revendication 4 dans lequel la fraction aqueuse obtenue à l'étape e) est au moins en partie recyclée à l'étape c) dans la zone d'extraction (L1) par lavage à l'eau.

6. Procédé selon l'une des revendications 2 à 5 dans lequel au moins une partie de la fraction (F) ou de la fraction légère sensiblement anhydre obtenue à l'étape e) à partir de ladite fraction (F) est au moins en partie envoyée dans une zone de craquage catalytique.

7. Procédé selon l'une des revendications 2 à 5 dans lequel au moins une partie de la fraction (F) ou de la fraction légère sensiblement anhydre obtenue à l'étape e) à partir de ladite fraction (F) est au moins en partie envoyée dans une zone de synthèse d'éther par réaction entre au moins une oléfine tertiaire et au moins un alcool.

8. Procédé selon l'une des revendications 1 à 7 comprenant une étape f) dans laquelle au moins une partie de la fraction (C) issue de l'étape c) est envoyée dans une zone de fractionnement (C3) à partir de laquelle on récupère une fraction (G) contenant la majeure partie de l'alcool initialement présent dans ladite partie et une fraction aqueuse (H) débarrassée de la majeure partie de l'alcool initialement présent dans ladite partie.

9. Procédé selon la revendication 8 dans lequel au moins une partie de la fraction (G) est envoyée dans une zone de synthèse d'éther par réaction entre au moins une oléfine tertiaire et au moins un alcool.

10. Procédé selon l'une des revendications 8 ou 9 dans lequel au moins une partie de la fraction aqueuse (H) est envoyée au moins en partie dans une section de traitement des eaux.

11. Procédé selon l'une des revendications 8 à 10 dans lequel au moins une partie de la fraction aqueuse (H) est au moins en partie recyclée à l'étape c) dans la zone (L1) d'extraction par lavage à l'eau.

12. Procédé selon l'une des revendications 1 à 11 dans lequel au moins une partie de la fraction (B) est envoyée dans une zone de synthèse d'éther par réaction entre au moins une oléfine tertiaire et au moins un alcool.

13. Procédé selon l'une des revendications 1 à 12 dans lequel au moins une partie de la fraction aqueuse (Le) obtenue à l'étape d) est au moins en partie recyclée à l'étape c) dans la zone d'extraction par lavage à l'eau.

14. Procédé selon l'une des revendications 1 à 13 dans lequel, à l'étape c) de purification d'au moins une partie de la fraction (A), on introduit dans la zone (L1) d'extraction par lavage à l'eau une quantité d'eau telle que le rapport volumique entre le volume de ladite quantité d'eau introduite dans ladite zone d'extraction et celui de ladite partie de fraction (A) (Vₑₐᵤ/V_{A}) est de 0,005 à 20.

15. Procédé selon l'une des revendications 1 à 14 dans lequel la zone (L1) d'extraction par lavage à l'eau de l'étape c) comporte au moins un moyen d'introduction d'eau d'appoint.

16. Procédé selon l'une des revendications 1 à 15 dans lequel le catalyseur de l'étape a) est choisi dans le groupe formé par les résines acides organiques et les résines acides minérales.

17. Procédé selon l'une des revendications 1 à 16 dans lequel le catalyseur de l'étape a) est choisi dans le groupe formé par les solides minéraux greffés comportant au moins un groupe organique de type alkyl-sulfonique, aryl-sulfonique ou alkylaryl-sulfonique.

18. Procédé selon l'une des revendications 1 à 17 dans lequel le catalyseur de l'étape a) est choisi dans le groupe formé par les polysiloxanes greffés par au moins un groupe alkyl-sulfonique.

## Patentansprüche

1. Verfahren zur Erzeugung tertiären Olefins durch Zersetzung von tertiärem Alkylether umfassend
a) eine Stufe der Zersetzung wenigstens eines tertiären Alkylethers in einer Reaktionszone, die wenigstens einen Reaktor (R1) umfasst, der über einen Katalysator zur Zersetzung dieses Ethers verfügt, wobei diese Stufe unter Bedingungen durchgeführt wird, welche die wenigstens teilweise Zersetzung dieses tertiären Alkylethers in ein Produkt ermöglicht, das wenigstens einen Alkohol und wenigstens ein tertiäres Olefin enthält,
b) eine Stufe zur Fraktionierung wenigstens eines Teils des aus der Stufe a) stammenden Produkts in einer Fraktionierungszone (C1), die es ermöglicht, einerseits eine Fraktion (A) zu erhalten, die den größeren Teil des tertiären Olefins und ggf. eine kleinere Fraktion von Alkohol und evtl. leichter Zusammensetzungen enthält und andererseits eine Fraktion (B), die den größeren Teil des in Stufe a) gebildeten Alkohols und ggf. in Stufe a) nicht zersetzten Ethers enthält.
c) eine Stufe zur Reinigung wenigstens eines Teils der Fraktion (A), in der dieser Teil in eine Extraktionszone (L1) mittels Waschen durch Wasser gegeben wird, aus der man eine wässrige Fraktion (C) erhält, welche den größeren Teil des ursprünglich in diesem Teil enthaltenen Alkohols und eine Fraktion (D) enthält, die den größeren Teil des ursprünglich in diesem Teil vorhandenen tertiären Olefins enthält, wobei diese Fraktion (D) dieses tertiäre Olefin, Wasser, ggf. leichte Verbindungen enthält und im wesentlichen frei von Alkohol ist, wobei sich dieses Verfahren dadurch auszeichnet, das es eine Stufe d) umfasst, in der wenigstens ein Teil der aus der Stufe (C) stammenden Fraktion (D) in eine Trennzone (Co) geschickt wird, aus der man eine wässrige flüssige Fraktion (Le) und eine flüssige Fraktion (Lc) gewinnt, welche den größeren Teil des tertiären, ursprünglich in der Fraktion (D) enthaltenen Olefins enthält, wobei diese Fraktion (Lc) dieses tertiäre Olefin, eine geringe Menge Wasser und ggf. leichte Zusammensetzungen enthält.

2. Verfahren nach Anspruch 1, bei dem wenigstens ein Teil der flüssigen, in Stufe d) gewonnenen Fraktion (Lc) in eine Stufe e) in eine Fraktionierungszone (C2) geschickt wird, in der dieser Teil der Fraktion (Lc) einerseits in eine Fraktion (E), die das tertiäre Olefin enthält und andererseits in eine Fraktion (F) fraktioniert wird, die den größeren Teil der leichten, evtl. vorhandenen Verbindungen und ggf. einen geringen Anteil von Restwasser enthält.

3. Verfahren nach Anspruch 2, bei dem die Fraktionierungszone der Stufe e) wenigstens ein Mittel umfasst, das es ermöglicht, aus der Fraktion (F) eine leichte, im wesentlichen wasserfreie Fraktion zu gewinnen.

4. Verfahren nach Anspruch 3, bei dem dieses Mittel einen Trennballon umfasst, der mit wenigstens einem Mittel ausgestattet ist, welches die Dekantierung und das Abziehen einer wässrigen Fraktion gestattet.

5. Verfahren nach Anspruch 4, bei dem die wässrige, in Stufe e) erhaltene Fraktion wenigstens zum Teil zur Stufe c) in der Extraktionszone (L1) durch Waschen mit Wasser rezykliert wird.

6. Verfahren nach einem der Ansprüche 2 bis 5, bei dem wenigstens ein Teil der Fraktion (F) oder der leichten, im wesentlichen wasserfreien, in Stufe e) aus dieser Fraktion (F) erhaltenen Fraktion wenigstens zum Teil in eine Zone des katalytischen Krackens geschickt wird.

7. Verfahren nach einem der Ansprüche 2 bis 5, bei dem wenigstens ein Teil der Fraktion (F) oder der leichten, im wesentlichen wasserfreien, in Stufe e) aus dieser Fraktion (F) erhaltenen Fraktion wenigstens zum Teil in eine Synthesezone für Ether gegeben wird und zwar durch Reaktion zwischen wenigstens einem tertiären Olefin und wenigstens einem Alkohol.

8. Verfahren nach einem der Ansprüche 1 bis 7, eine Stufe f) umfassend, in der wenigstens ein Teil der aus der Stufe c) stammenden Fraktion (C) in eine Fraktionierungszone (C3) gegeben wird, aus der man eine Fraktion (G) gewinnt, welche den größeren Teil des ursprünglich in diesem Teil enthaltenen Alkohols und eine wässrige Fraktion (H) enthält, die vom Hauptteil des ursprünglich in diesem Teil enthaltenen Alkohols befreit ist.

9. Verfahren nach einem Anspruch 8, bei dem wenigstens ein Teil der Fraktion (G) in eine Ethersynthesezone durch Reaktion zwischen wenigstens einem tertiären Olefin und wenigstens einem Alkohol geschickt wird.

10. Verfahren nach einem der Ansprüche 8 oder 9, bei dem wenigstens ein Teil der wässrigen Fraktion (H) wenigstens zum Teil in einen Abschnitt zur Wasserbehandlung gegeben wird.

11. Verfahren nach einem der Ansprüche 8 bis 10, bei dem wenigstens ein Teil der wässrigen Fraktion (H) wenigstens zum Teil zur Stufe c) in der Extraktionszone (L1) für das Waschen mit rezykliert wird.

12. Verfahren nach einem der Ansprüche 1 bis 11, bei dem wenigstens ein ,Teil der Fraktion (B) in eine Ethersynthesezone durch Reaktion zwischen wenigstens einem tertiären Olefin und wenigstens einem Alkohol geschickt wird.

13. Verfahren nach einem der Ansprüche 1 bis 12, bei dem wenigstens ein Teil der wässrigen, in Stufe d) erhaltenen Fraktion (Le) wenigstens zum T eil zur Stufe c) in die Extraktionszone zum Waschen mit Wasser rezykliert wird.

14. Verfahren nach einem der Ansprüche 1 bis 13, bei dem in Stufe c) zur Reinigung wenigstens eines Teils der Fraktion (A), die man in die Zone L1 zur Extraktion für das Waschen mit Wasser eine Wassermenge derart einführt, dass das Volumenverhältnis zwischen dem Volumen zwischen der in dieser Extraktionszone eingeführten Wassermenge und der des Teils der Fraktion (A) (V_{Wasser}/Vₐ) zwischen 0,005 und 20 liegt.

15. Verfahren nach einem der Ansprüche 1 bis 14, bei dem die Zone (L1) der Extraktion für das Waschen mit Wasser in Stufe c) wenigstens ein Mittel zur Wasserzufuhr umfasst.

16. Verfahren nach einem der Ansprüche 1 bis 15, bei dem der Katalysator der Stufe a) gewählt ist aus der durch die organischen sauren Harze und die mineralischen sauren Harze gebildeten Gruppe.

17. Verfahren nach einem der Ansprüche 1 bis 16, bei dem der Katalysator der Stufe a) gewählt ist aus der durch die mineralischen gepfropften Feststoffe gebildeten Gruppe, die wenigstens eine organische Gruppe vom Alkyl-Sulfontyp, Aryl-Sulfontyp oder Alkylaryl-Sulfontyp umfasst.

18. Verfahren nach einem der Ansprüche 1 bis 17, bei dem der Katalysator der Stufe a) aus der Gruppe gewählt ist, die durch die gepfropften Polysiloxane gebildet wird, welche durch wenigstens eine Alkyl-Sulfongruppe gepfropft sind.

## Claims

1. A process for the production of a tertiary olefin by decomposing a tertiary alkyl ether, comprising:
a) a step for decomposing at least one tertiary alkyl ether in a reaction zone comprising at least one reactor (R1) containing a catalyst for decomposing said ether, said step being carried out under conditions which can at least partially decompose said tertiary alkyl ether to a product containing at least one alcohol and at least one tertiary olefin;
b) a step for fractionating at least a portion of the product from step a) in a fractionation zone (C1) to obtain a fraction (A) containing the major portion of the tertiary olefin and possibly a minor fraction of alcohol and any light compounds, and a fraction (B) containing the major portion of the alcohol formed in step a) and possibly ether which has not been decomposed in step a);
c) a step for purifying at least a portion of fraction (A) in which said portion is sent to a water washing extraction zone (L1) from which an aqueous fraction (C) is obtained containing the major portion of the alcohol initially present in said portion and a fraction (D) containing the major portion of the tertiary olefin initially present in said portion, said fraction (D) containing said tertiary olefin, water and possibly light compounds and being substantially free of alcohol;
said process being characterized in that it comprises a step d) in which at least a portion of fraction (D) from step c) is sent to a separation zone (Co) from which an aqueous liquid fraction (Le) and a liquid fraction (Lc) containing the major portion of the tertiary olefin initially present in fraction (D) are obtained, said fraction (Lc) containing said tertiary olefin, a small quantity of water and possibly light compounds.

2. A process according to claim 1, in which at least a portion of fraction (Lc) recovered from step d) is sent to a step e) in a fractionation zone (C2) in which said portion of fraction (Lc) is fractionated into a fraction (E) containing the tertiary olefin and a fraction (F) containing the major portion of any light compounds and possibly a small quantity of residual water.

3. A process according to claim 2, in which the fractionation zone of step e) comprises at least one means for recovering a substantially anhydrous light fraction from fraction (F).

4. A process according to claim 3, in which said means comprises a separator drum provided with at least one means for decanting and extracting an aqueous fraction.

5. A process according to claim 4, in which at least part of the aqueous fraction obtained from step e) is recycled to step c) in the water washing extraction zone (L1).

6. A process according to any one of claims 2 to 5, in which at least a portion of fraction (F) or the substantially anhydrous light fraction obtained from step e) from said fraction (F) is sent at least in part to a catalytic cracking zone.

7. A process according to any one of claims 2 to 5, in which at least a portion of fraction (F) or the substantially anhydrous light fraction obtained from step e) from said fraction (F) is sent at least in part to a zone for synthesising an ether by reaction between at least one tertiary olefin and at least one alcohol.

8. A process according to any one of claims 1 to 7, comprising a step f) in which at least a portion of fraction (C) from step c) is sent to a fractionation zone (C3) from which a fraction (G) containing the major portion of the alcohol initially present in said portion and also an aqueous fraction (H) which is free of the major portion of the alcohol initially present in said portion are recovered.

9. A process according to claim 8, in which at least a portion of fraction (G) is sent to a zone for synthesising an ether by reaction between at least one tertiary olefin and at least one alcohol.

10. A process according to claim 8 or claim 9, in which at least a portion of the aqueous fraction (H) is sent at least in part to a water treatment section.

11. A process according to any one of claims 8 to 10, in which at least a portion of the aqueous fraction (H) is recycled at least in part to step c) in the water washing extraction zone (L1).

12. A process according to any one of claims 1 to 11, in which at least a portion of fraction (B) is sent to a zone for synthesising an ether by reaction between at least one tertiary olefin and at least one alcohol.

13. A process according to any one of claims 1 to 12, in which at least a portion of the aqueous fraction (Le) obtained in step d) is recycled at least in part to step c) in the water washing extraction zone.

14. A process according to any one of claims 1 to 13 in which, in step c) for purifying at least a portion of the fraction (A), a quantity of water is introduced into the water washing extraction zone (L1) such that the ratio between the volume of said quantity of water introduced into said extraction zone and that of said portion of fraction (A) (V_{water}/V_{A}) is 0.005 to 20 by volume.

15. A process according to any one of claims 1 to 14, in which the water washing extraction zone (L1) in step c) comprises at least one means for introducing makeup water.

16. A process according to any one of claims 1 to 15, in which the catalyst of step a) is selected from the group formed by organic acid resins and mineral acid resins.

17. A process according to any one of claims 1 to 16, in which the catalyst of step a) is selected from the group formed by grafted mineral solids comprising at least one organic alkylsulphonic, arylsulphonic or alkylarylsulphonic type group.

18. A process according to any one of claims 1 to 17, in which he catalyst in step a) is selected from the group formed by polysiloxanes grafted with at least one alkylsulphonic group.
